# EUROPEAN PATENT SPECIFICATION

(11) **EP 3 538 060 B1**
(45) Date of publication and mention of the grant of the patent: **22.04.2020**
(21) Application number: 17777283.7
(22) Date of filing: 03.10.2017
(51) Int. Cl.: A61K 8/29, A61K 8/36, A61Q 15/00, A61K 8/19

(54) **AN ANTIPERSPIRANT COMPOSITION**
SCHWEISSHEMMENDE ZUSAMMENSETZUNG
COMPOSITION ANTI TRANSPIRATION

(30) Priority: 09.11.2016 WO PCT/CN2016/000620; 22.12.2016 EP 16206108
(43) Date of publication of application: 18.09.2019
(73) Proprietor: Unilever N.V., 3013 AL Rotterdam (NL); Unilever PLC, London, Greater London EC4Y 0DY (GB)
(72) Inventor: LIU, Renjiang, Shanghai 200335 (CN); WANG, Jinfang, Shanghai 200335 (CN); WATERFIELD, Philip, Christopher, Wirral Merseyside CH63 3JW (GB); ZHOU, Huanjun, Shanghai 200335 (CN)
(74) Representative: Whaley, Christopher
(86) International application number: PCT/EP2017/075075
(87) International publication number: WO 2018/086797

(56) References cited:
- GB-A- 2 299 506

## Description

### Field of the invention

The present invention is in the field of compositions comprising antiperspirant actives, particularly cosmetic compositions. The present invention more particularly relates to compositions comprising titanium based antiperspirant actives which are stable before use and effective in ensuring anti perspirancy for a long period of time after the product is applied on to the body by the consumer.

### Background of the invention

The present invention relates to compositions, such as those that contain antiperspirant actives. These actives are added to compositions to reduce perspiration on application to the surface of the body, particularly to the underarm regions of the human body viz. the axilla. Antiperspirant actives are typically astringent metal salts such as those of aluminium or zirconium salts. Antiperspirant actives are usually incorporated in compositions at low pH, in the range of 2 to 7. It has also been proposed in the past that salts of titanium may be used but these have not been commercially exploited so far due to technical difficulties in formulating as well as delivering them to have sufficient sustained efficacy when in use.

US3090728 (Carter Products, 1963) discloses an antiperspirant composition comprising an oil-water emulsion vehicle having dissolved therein an antiperspirant agent selected from the group consisting of titanic acid complexes of hydroxy aliphatic carboxylic acids having from 2 to 6 carbon atoms and mixtures thereof, said composition having a pH in the range of about 2.5 to 5.

EP0586235 (Unilever, 1992) discloses an antiperspirant composition suitable for topical application to the human skin, comprising an effective amount of a titanium salt of a hydroxy aliphatic carboxylic acid having a chain length of from C2 to C10, the composition having a pH in aqueous solution of greater than 5.

GB2299506 (Unilever, 1996) discloses an antiperspirant composition for topical application to the human skin, comprising effective amount of a titanium salt selected from the group consisting of a straight or branched chain saturated or unsaturated non-hydroxy C₂-C₂₀ carboxylic acid, C₇-C₁₂ aromatic carboxylic acids, ethanolamine complexes, and C₄-C₁₂ aliphatic polyols, in a cosmetically suitable vehicle.

The present inventors have tested the titanium salts proposed in the past for anti-perspirancy benefits and discovered that they suffer either from being unstable when formulated or do not provide the desired benefit for the long period of time after application on the body as desired by the consumer, or both of these problems. It is desired that the antiperspirant active does not degrade when formulated and stored for periods of time (sometimes up to several months) before they are used by the consumer. When the formulated product is applied to the skin it is desired that the antiperspirant active hydrolyses in the aqueous saline media of sweat to form particles of the right size such that it can clog or gel in the sweat pores and inhibit further sweating. This rate of hydrolysis to form the particles should not be too fast, else the efficacy is experienced by the consumer only for a few seconds or minutes. It is desirable that the antiperspirant benefit is experienced over several hours. Thus the hydrolysis kinetics should be such as to enable the reaction to occur slowly over a matter of several hours. It should also not be so slow that only a small portion of the antiperspirant active is utilized and the rest is washed away when the consumer has a bath or a shower. The present inventors then found out that this problem can be solved by formulating a specific titanium organo salt with two different types of acids in a topically acceptable carrier.

It is thus an object of the present invention to provide for a antiperspirant composition which exhibits sustained antiperspirant efficacy from titanium based actives.

### Summary of the invention

According to the first aspect of the present invention there is provided an antiperspirant composition with a pH in the range of 3 to 7 comprising:
(i) a titanium compound chelated by alkanolamine having the chemical formula:

   TiNₐ(NH)_{b}(NH₂)_{c}O_{d}(OH)ₑ(CH₂)ₘ(CH₃)ₙ Formula 1

   where a, b and c each independently have a value of from 0 to 3;
   d and e each independently have a value from 0 to 10;
   m has a value from 1 to 18;
   n has a value from 0 to 9;
   wherein a+b+c has a value from 1 to 3; d+e has a value from 4 to 10 and m+n has a value from 1 to 18;
(ii) a mineral acid;
(iii) a carboxylic acid comprising at least two groups, one of which is a carboxylic acid group and the others may be a carboxylic acid group or a hydroxyl group; or a salt thereof and
(iv) a topically acceptable carrier,
wherein the mole ratio of the titanium compound to carboxylic acid is in the range of 1:0.01 to 1:5.

It is particularly preferred that the ligands present in the titanium organosalt are selected from triethanolamine, diethanolamine or monoethanolamine, most preferably trioethanol amine. Preferred titanium compounds have the following structures.

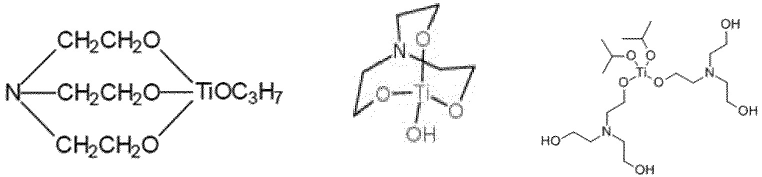

It is particularly preferred that the pH of the composition is from 4 to 7.

According to another aspect of the present invention there is provided a method of reducing perspiration comprising the step of applying a composition as claimed in the first aspect on to the desired skin surface.

According to another aspect of the present invention there is provided non-therapeutic use of a titanium compound of formula 1 for reduction of sweat or as an antiperspirant active.

According to another aspect of the present invention, there is provided a titanium compound of formula 1 for the treatment of excessive sweating.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The compositions of the invention are typically "personal care compositions", suitable for cosmetic use as detailed below. Further, use of the compositions of the invention is typically cosmetic, non-therapeutic use.

In some embodiments of the present invention, the compositions may be used for the therapeutic treatment of hyperhidrosis (extreme sweating).

By "A Personal Care Composition" as used herein, is meant to include a composition for topical application to the skin of mammals, especially humans. Such a composition is preferably of the leave-on type. By a leave-on composition is meant a composition that is applied to the desired skin surface and left on for a period of time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. It is especially useful for delivering low pH compositions to the axilla of an individual for anti-perspirancy benefits. "Skin" as used herein is meant to include skin on any part of the body (e.g., neck, chest, back, arms, underarms, hands, legs, buttocks and scalp) especially the underarms.

The present invention is directed to delivering a titanium based antiperspirant active on to the topical surface of a human body. The active for inclusion in the composition is a titanium compound chelated by alkanolamine having the chemical formula 1,

TiNₐ(NH)_{b}(NH₂)_{c}O_{d}(OH)ₑ(CH₂)ₘ(CH₃)ₙ

where a, b and c each independently have a value of from 0 to 3;
d and e each independently have a value from 0 to 10;
m has a value from 1 to 18;
n has a value from 0 to 9;
and wherein a+b+c has a value from 1 to 3; d+e has a value from 4 to 10 and m+n has a value from 1 to 18.

The titanium organo salt is preferably a titanium alkanolamine complex wherein the ligands is preferably selected from a triethanolamine, a diethanolamine or a monoethanolamine.

The most preferred compound for use in the present invention is Titanium 2,2,2 - nitrilotrisethanolate (CAS number: 15879-01-3) having the following structure:

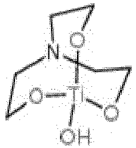

Titanium 2,2,2 - nitrilotrisethanolate, (having CAS number: 15879-01-3) is the most preferred titanium alkanolamine chelated complex, as per this invention (compound of formula 2). It is usually supplied dispersed in isopropanol as thick light yellowish liquid. One major supplier is Dorf Ketal. The aqueous dilution is alkaline due to the presence of triethanolamine. In the chemical industry, it has normally been used as a crosslinker, a catalyst, or a coating adhesion enhancer.

The above ttitanium compound (of formula 1) is preferably included in 1 to 30%, more preferably 1 to 15%, further more preferably 5 to 10% by weight of the composition.

The composition comprises both a mineral acid and a carboxylic acid or a salt thereof meeting the claimed specification. Without wishing to be bound by theory the inventors believe that there are two steps necessary to ensure the long lasting benefits afforded by the present invention. It is essential to have the composition formulated and delivered at a low pH in the range of 3 to 7 preferably 4 to 7 further more preferably 5 to 7 in order for the formulation to be stable and effective. This is ensured by including therein strong mineral acids at low concentration so that the desired pH of the composition is quickly attained when the composition is formulated and further when the composition is diluted in the presence of sweat. Once inside the sweat pores, possibly the titanium complex crosslinks with the mucin or other components inside the pores thereby forming a gel network which clogs the pores of the sweat glands thereby ensuring antiperspirant benefit for a long period of time.

The mineral acid for inclusion in the composition is preferably selected from hydrochloric acid, nitric acid, phosphoric acid or sulphuric acid, more preferably hydrochloric acid or nitric acid. The mineral acid may be included in 0.01 to 7%, preferably from 0.1 to 1.5% by weight of the composition.

The composition of the invention comprises a carboxylic acid comprising at least two groups, one of which is a carboxylic acid group and the others may be a carboxylic acid group or a hydroxyl group; or a salt thereof. Thus it is essential that of the various pendant groups in the carboxylic acid (of which there must be at least two), one is necessarily a carboxylic acid group. If there are two pendant groups, the second group may be a carboxylic acid group or a hydroxyl group. If there are three groups, all three could be carboxylic groups or it is possible that two of the groups are carboxylic acid groups and one is a hydroxyl group or it is possible that one is a carboxylic acid group and two are hydroxyl groups. And so on.

Based on the above criteria the preferred carboxylic acids are oxalic acid, malonic acid, adipic acid, maleic acid, fumaric acid, glycolic acid, lactic acid, salicylic acid, citric acid, tartaric acid, gluconic acid, or malic acid. Particularly preferred carboxylic acids are tartaric acid, oxalic acid, lactic acid or citric acid.

The various carboxylic acids (as above) which are found to be effective for inclusion in the present invention have the structures as given below:

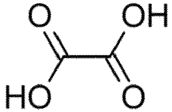

Oxalic acid (Two carboxylic acid groups)

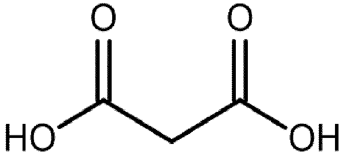

Malonic acid (Two carboxylic acid groups)

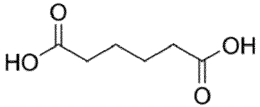

Adipic acid (Two carboxylic acid groups)

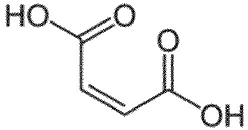

Maleic acid (Two carboxylic acid groups)

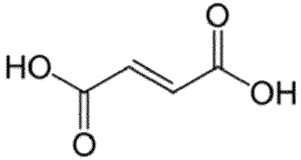

Fumaric acid (Two carboxylic acid groups)

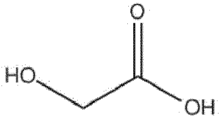

Glycolic acid (One carboxylic acid group and one hydroxyl group)

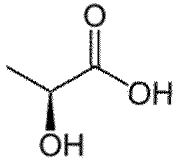

Lactic acid (One carboxylic acid group and one hydroxyl group)

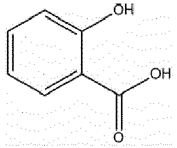

Salicylic acid (One carboxylic acid group and one hydroxyl group)

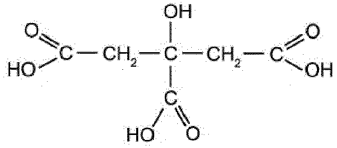

Citric acid (Three carboxylic acid groups and one hydroxyl group)

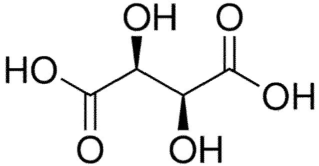

Tartaric acid (Two carboxylic acid groups and two hydroxyl groups)

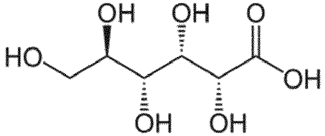

Gluconic acid (One carboxylic acid and five hydroxyl groups)

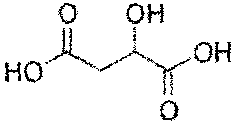

Malic acid (Two carboxylic acid groups and one hydroxyl group)

The salt of the carboxylic acids may be the alkali metal or alkaline earth metal salt, the most preferred salt being alkali metal salt.

Further, certain carboxylic acids which do not meet the above criteria were tried and found to be not suitable for meeting the objects of the present invention.

Some of those acids (which are unsuitable for use in the present invention) and their structures are given below:

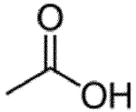

Acetic acid (Only one carboxylic acid)

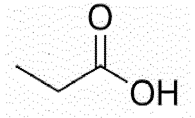

Propionic acid (Only one carboxylic acid)

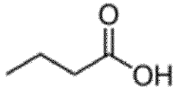

Butyric acid (Only one carboxylic acid)

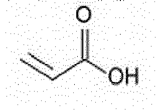

Acrylic acid (Only one carboxylic acid)

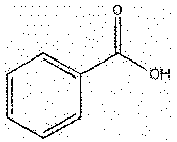

Benzoic acid (Only one carboxylic acid)

Carboxylic acid or salt thereof, as per the invention, is preferably included in 0.01 to 20%, more preferably 0.03 to 16% by weight of the composition.

The mole ratio of the titanium compound to carboxylic acid is in the range of 1:0.01 to 1:5, preferably 1:0.03 to 1:1. It is also particularly preferred that the mole ratio of the titanium compound to the mineral acid is in the range of 1:0.01 to 1:10, preferably 1:0.03 to 1:1. If the carboxylic acid level is too high, the composition is too stable to be hydrolysed by sweat; if the mineral acid level is too high, the composition has a tendency to destabilise in a few weeks.

Antiperspirant compositions of the present invention may advantageously comprise an additional, non-titanium, antiperspirant active. Whilst this might be a conventional antiperspirant salt comprising Al and/or Zr, such as aluminium chlorohydrate or aluminium-zirconium chlorohydrate optionally complexed with glycine, it is preferred that any additional antiperspirant active is not of this type.

Other components commonly included in conventional antiperspirant compositions may also be incorporated in the compositions of the present invention. Such components include skin care agents such emollients, humectants and skin barrier promoters; skin appearance modifiers such as skin lightening agents and skin smoothing agents; anti-microbial agents, in particular organic anti-microbial agents, and preservatives.

The anti-perspirant active can be applied cosmetically and topically to the skin, broadly speaking, by one of two methods. Different consumers prefer one method or the other. In one method, sometimes called a contact method, a composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of the dispenser, such as a pump or a squeezable sidewall or by internally generated pressure arising from a fraction of a liquefied propellant volatilizing, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

### Stick or soft solid compositions

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

### Aerosol compositions

The antiperspirant composition may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquefied by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax. When the composition comprises a propellant it is delivered as an aerosol.

The composition of the present invention can comprise a wide range of other optional components. The CTFA Personal Care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

The present invention also provides for a method of reducing perspiration comprising the step of applying the composition of the first aspect on to the desired skin surface. The skin surface could be any topical surface which is prone to sweating especially the axilla i.e. the underarm portion of the human body. The method is preferably non-therapeutic. The invention also provides for use of the titanium compound having the chemical formula 1, for manufacture of a composition as claimed in the present invention, for reduction of sweat.

The invention also provides for use of a titanium compound according Formula 1 as an antiperspirant active.

The invention will now be demonstrated with the help of the following non-limiting examples.

### Examples

### Examples A-D and 1-7: Stability of compositions at room temperature over one week storage.

The following composition were prepared as given in Table - 1

**Table - 1**

| Example | Titanium compound^{A}, wt% | Carboxylic Acid | Carboxylic acid, wt% | Mineral acid | Mineral Acid, Wt% |
|---|---|---|---|---|---|
| A | 5.0 | - | - | HCl | 0.10 |
| B | 5.0 | Tartaric | 1.0 | - | - |
| C | 5.0 | Acetic | 1.0 | - | - |
| D | 5.0 | Vanillic | 1.0 | - | - |
| 1 | 5.0 | Tartaric | 0.66 | HCl | 0.21 |
| 2 | 5.0 | Tartaric | 0.33 | HCl | 0.33 |
| 3 | 5.0 | Tartaric | 0.33 | HCl | 0.51 |
| 4 | 5.0 | Tartaric | 0.33 | HNO₃ | 0.58 |
| 5 | 5.0 | Citric | 0.20 | HCl | 0.41 |
| 6 | 5.0 | Oxalic | 0.45 | HCl | 0.29 |
| 7 | 5.0 | Lactic | 0.60 | HCl | 0.32 |

| | | | | | |
|---|---|---|---|---|---|
| ^{A} The titanium compound used was the compound of formula 2. | | | | | |

The above compositions were tested for stability by using the following procedure: 2 g of the titanium compound was weighed then added into 30 ml of de-ionised water in a beaker and stirred well. Certain amount of carboxylic acids (according to the dosage in Table 1) were added dropwise with stirring. Afterwards, required dose (according to Table 1) of mineral acids were added dropwise slowly. The rest of the water was added to reach a total of 40 g system. The composition was further stirred for another 10 minutes.

The samples were transferred into transparent plastic jars which were then stored at room temperature (25 °C). The appearance of each of the samples was checked visually and recorded at various time points. The samples were graded as either Transparent (T), Cloudy (C) or Precipitate (P). The data on the appearance of the samples is summarized in Table - 2 below:

**Table - 2**

| Example | 0 hr | 2 hr | 24 hr | 3 days | 7 days |
|---|---|---|---|---|---|
| A | C | C | C | P | P |
| B | T | T | T | T | T |
| C | C | C | C | P | P |
| D | C | C | P | P | P |
| 1 | T | T | T | T | T |
| 2 | T | T | T | T | T |
| 3 | T | T | T | T | T |
| 4 | T | T | T | T | T |
| 5 | T | T | T | T | T |
| 6 | T | T | T | T | T |
| 7 | T | T | T | T | T |

The data in Table - 2 above indicates that compositions as per the invention (Examples 1- 7) were stable on storage for up to 1 week while those outside the invention A, C, and D were unstable right at the time of preparation. Sample B was stable but was found to be unsuitable for use in the present invention as will be apparent from the data in Table 3 below.

### Examples B and 2 to 7: Hydrolysis of the compositions in the presence of salt

It is desirable that the compositions are stable when stored but hydrolyse to form an insoluble complex soon (in matter of minutes or hours) after they come in contact with sweat on the human body. These particulates would then be expected to clog or gel in the sweat pores thereby acting as antiperspirants.

The following procedure was used to check the hydrolysis of the various compositions:
0.9 g of sodium chloride was added to 10ml composition system (9%wt) to allow hydrolysis to start. The samples were transferred into transparent plastic jars which were then stored at room temperature (25 °C). The appearance of each of the samples was checked visually and recorded at various time points. The samples were graded as either Transparent (T), Cloudy (C) or Precipitate (P). The data on the appearance of the samples is summarized in Table - 3 below:

**Table -3**

| Example | 0 hr | 2 hr | 24 hr | 3 days | 7 days |
|---|---|---|---|---|---|
| B | T | T | T | T | T |
| 2 | T | T | C | C | P |
| 3 | T | C | C | P | P |
| 4 | T | T | C | C | P |
| 5 | T | T | C | C | C |
| 6 | T | T | C | C | P |
| 7 | T | T | C | C | C |

The data in Table - 3 above indicates that compositions as per the invention 2 to 7 are transparent on application but progressively get cloudy or precipitate in reasonable time frame of use of the product on the human body (from a few hours to days). Composition as per example B (outside the invention) is unsuitable as no such cloudiness or precipitations is seen even after one week.

### Examples E. 2, 8, 9: Quantification of cloudiness by measurement of the particles size

The cloudiness of the composition as per the invention was quantified by measuring the increase of particle size with time and the data for the various compositions is given below. Table - 4 lists the various compositions and Table -5 the measured particle size. The particle size was measured using the following procedure:
DLS (Dynamic light scattering) Particle size tracking: The compositions were diluted four times to give better DLS signaling and 0.09g NaCl was added to 10ml composition system before the test (0.9% wt NaCl). The samples were transferred into disposable sizing cuvette and placed into DLS Nanosizer (Malvern Zetasizer Nano ZS). The mean volume size of the titanium dioxide particles were measured with at least two repeats at each time point at room temperature (25 °C).

**Table - 4**

| Example | Titanium compound^{A}, wt% | Carboxylic Acid | Carboxylic acid, wt% | Mineral acid | Mineral Acid, Wt% |
|---|---|---|---|---|---|
| E | 5.0 | - | - | - | - |
| 2 | 5.0 | Tartaric | 0.33 | HCl | 0.33 |
| 8 | 5.0 | Tartaric | 0.26 | HCl | 0.38 |
| 9 | 5.0 | Tartaric | 0.19 | HCl | 0.39 |

**Table - 5**

| | Volume mean (nm) | | | |
|---|---|---|---|---|
| Example | 0 hr | 3 hr | 5 hr | 24 hr |
| E | 2.0 | 66.0 | 2735 | ND |
| 2 | 3.0 | 4.9 | 5.1 | 8.1 |
| 8 | 4.6 | 5.6 | 6.6 | 10.3 |
| 9 | 6.7 | 13.5 | 20.8 | 70.6 |

The data in Table -5 above indicates that for compositions as per the invention (Examples 2, 8, and 9) the particle size increases fairly slowly with time thereby ensuring that the compositions will be effective in the time frames of use by a consumer for anti-perspirancy benefit.

### Example 10: A Roll-on formulation

**Table - 6**

| Ingredients | Dosage (%) |
|---|---|
| Compound of formula 2 | 5.0 |
| Tartaric acid | 0.33 |
| HCl | 0.15 |
| Natrosol 250HHR | 1.0 |
| H₂O | Up to 100 |

Natrosol 250HHR is a hydroxyethylcellulose and is included here as a thickener. The above formulation was stored for over four weeks at room temperature and was found to be stable.

## Claims

1. An antiperspirant composition with a pH in the range of 3 to 7 comprising
(i) A titanium compound chelated by alkanolamine having the chemical formula:
TiNₐ(NH)_{b}(NH₂)_{c}O_{d}(OH)ₑ(CH₂)ₘ(CH₃)ₙ Formula 1
where a, b and c each independently have a value of from 0 to 3;
d and e each independently have a value from 0 to 10;
m has a value from 1 to 18;
n has a value from 0 to 9;
wherein a+b+c has a value from 1 to 3; d+e has a value from 4 to 10 and m+n has a value from 1 to 18;
(ii) a mineral acid;
(iii) a carboxylic acid comprising at least two groups, one of which is a carboxylic acid group and the others may be a carboxylic acid group or a hydroxyl group; or a salt thereof and
(iv) a topically acceptable carrier,
wherein the mole ratio of the titanium compound to carboxylic acid is in the range of 1:0.01 to 1:5.

2. A composition as claimed in claim 1 wherein the titanium compound is Titanium 2,2,2 - nitrilotrisethanolate (CAS number: 15879-01-3) having the following structure:

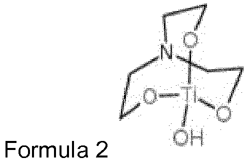

3. A composition as claimed in claim 1 or 2 wherein the mineral acid is selected from hydrochloric acid, nitric acid, phosphoric acid or sulphuric acid.

4. A composition as claimed in any one of the preceding claims wherein the mineral acid is included in an amount of 0.01 to 7% by weight of the composition.

5. A composition as claimed in any one of the preceding claims wherein the carboxylic acid is selected from oxalic acid, malonic acid, adipic acid, maleic acid, fumaric acid, glycolic acid, lactic acid, salicylic acid, citric acid, tartaric acid, gluconic acid, or malic acid.

6. A composition as claimed in claim 5 wherein the carboxylic acid is selected from tartaric acid, oxalic acid, lactic acid or citric acid.

7. A composition as claimed in any one of the preceding claims wherein the titanium compound is included in 1 to 30% by weight of the composition.

8. A composition as claimed in any one of the preceding claims wherein the mole ratio of the titanium compound to the mineral acid is in the range of 1: 0.1 to 1:10.

9. A composition as claimed in any one of the preceding claims wherein the topically acceptable carrier comprises a hydrophobic carrier or an aqueous carrier.

10. A composition as claimed in claim 9 wherein the hydrophobic carrier comprises a silicone compound, low boiling alcohol or a wax.

11. A composition as claimed in claim 9 or 10 which is delivered as a roll-on, a firm solid or a stick.

12. A composition as claimed in any one of the preceding claims 1 to 8 wherein the topically acceptable carrier comprises a propellant and the composition is delivered as an aerosol.

13. A method of reducing perspiration comprising the step of applying a composition as claimed in any one of the preceding claims on to the desired skin surface.

14. Non-therapeutic use of a titanium compound chelated by alkanolamine having the chemical formula:
TiNₐ(NH)_{b}(NH₂)_{c}O_{d}(OH)ₑ(CH₂)ₘ(CH₃)ₙ Formula 1
where a, b and c each independently have a value of from 0 to 3;
d and e each independently have a value from 0 to 10;
m has a value from 1 to 18;
n has a value from 0 to 9;
wherein a+b+c has a value from 1 to 3; d+e has a value from 4 to 10 and m+n has a value from 1 to 18,
as an antiperspirant active.

## Patentansprüche

1. Schweißhemmende Zusammensetzung mit einem pH-Wert in dem Bereich von 3 bis 7, umfassend
(i) eine Titanverbindung, chelatiert mit Alkanolamin, die die chemische Formel:
TiNₐ(NH)_{b}(NH₂)_{c}O_{d}(OH)ₑ(CH₂)ₘ(CH₃)ₙ Formel 1
aufweist,
worin a, b und c, jedes unabhängig, einen Wert von 0 bis 3 haben;
d und e, jedes unabhängig, einen Wert von 0 bis 10 haben;
m einen Wert von 1 bis 18 hat;
n einen Wert von 0 bis 9 hat;
wobei a+b+c einen Wert von 1 bis 3 hat; d+e einen Wert von 4 bis 10 hat und
m+n einen Wert von 1 bis 18 hat;
(ii) eine Mineralsäure;
(iii) eine Carbonsäure, die mindestens zwei Gruppen umfasst, von welchen eine eine Carbonsäuregruppe ist und die anderen eine Carbonsäuregruppe oder eine Hydroxylgruppe sein können; oder ein Salz davon und
(iv) einen topisch verträglichen Träger,
wobei das Molverhältnis der Titanverbindung zur Carbonsäure in dem Bereich von 1:0,01 bis 1:5 liegt.

2. Zusammensetzung, wie im Anspruch 1 beansprucht, wobei die Titanverbindung Titan-2,2,2-nitrilotrisethanolat (CAS-Nummer: 15879-01-3) darstellt und die folgende Strukturformel 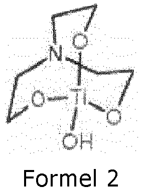 aufweist.

3. Zusammensetzung, wie im Anspruch 1 oder 2 beansprucht, wobei die Mineralsäure unter Salzsäure, Salpetersäure, Phosphorsäure oder Schwefelsäure ausgewählt ist.

4. Zusammensetzung, wie in irgendeinem der vorhergehenden Ansprüche beansprucht, wobei die Mineralsäure mit einer Menge von 0,01 bis 7 Gewichts-% der Zusammensetzung einbezogen ist.

5. Zusammensetzung, wie in irgendeinem der vorhergehenden Ansprüche beansprucht, wobei die Carbonsäure unter Oxalsäure, Malonsäure, Adipinsäure, Maleinsäure, Fumarsäure, Glykolsäure, Milchsäure, Salicylsäure, Zitronensäure, Weinsäure, Gluconsäure oder Äpfelsäure ausgewählt ist.

6. Zusammensetzung, wie im Anspruch 5 beansprucht, wobei die Carbonsäure unter Weinsäure, Oxalsäure, Milchsäure oder Zitronensäure ausgewählt ist.

7. Zusammensetzung, wie in irgendeinem der vorhergehenden Ansprüche beansprucht, wobei die Titanverbindung mit 1 bis 30 Gewichts-% der Zusammensetzung einbezogen ist.

8. Zusammensetzung, wie in irgendeinem der vorhergehenden Ansprüche beansprucht, wobei das Molverhältnis der Titanverbindung zu der Mineralsäure in dem Bereich von 1:0,1 bis 1:10 liegt.

9. Zusammensetzung, wie in irgendeinem der vorhergehenden Ansprüche beansprucht, wobei der topisch verträgliche Träger einen hydrophoben Träger oder einen wässrigen Träger umfasst.

10. Zusammensetzung, wie im Anspruch 9 beansprucht, wobei der hydrophobe Träger eine Siliconverbindung, einen niedrig siedenden Alkohol oder ein Wachs umfasst.

11. Zusammensetzung, wie im Anspruch 9 oder 10 beansprucht, die als Roll-on, als steifer Feststoff oder als Stift bereitgestellt ist.

12. Zusammensetzung, wie in irgendeinem der vorhergehenden Ansprüche 1 bis 8 beansprucht, wobei der topisch verträgliche Träger ein Treibmittel umfasst und die Zusammensetzung als Aerosol bereitgestellt wird.

13. Verfahren zum Vermindern der Perspiration, umfassend den Schritt des Aufbringens einer Zusammensetzung, wie in irgendeinem der vorhergehenden Ansprüche beansprucht, auf die gewünschte Hautoberfläche.

14. Nicht-therapeutische Verwendung einer Titanverbindung, die mit Alkanolamin chelatiert ist, die die chemische Formel:
TiNₐ(NH)_{b}(NH₂)_{c}O_{d}(OH)ₑ(CH₂)ₘ(CH₃)ₙ Formel 1
aufweist,
worin a, b und c, jedes unabhängig, einen Wert von 0 bis 3 haben;
d und e, jedes unabhängig, einen Wert von 0 bis 10 haben;
m einen Wert von 1 bis 18 hat;
n einen Wert von 0 bis 9 hat;
wobei a+b+c einen Wert von 1 bis 3 hat; d+e einen Wert von 4 bis 10 hat und m+n einen Wert von 1 bis 18 hat,
als schweißhemmendes Mittel.

## Revendications

1. Composition d'antiperspirant avec un pH dans l'intervalle de 3 à 7 comprenant
(i) un composé de titane chélaté par une alcanolamine présentant la formule chimique :
TiNₐ(NH)_{b}(NH₂)_{c}O_{d}(OH)ₑ(CH₂)ₘ(CH₃)ₙ Formule 1
où a, b et c présentent chacun indépendamment une valeur de 0 à 3 ;
d et e présentent chacun indépendamment une valeur de 0 à 10 ;
m présente une valeur de 1 à 18 ;
n présente une valeur de 0 à 9 ;
dans laquelle a+b+c présente une valeur de 1 à 3 ;
d+e présente une valeur de 4 à 10 et m+n présente une valeur de 1 à 18 ;
(ii) un acide minéral ;
(iii) un acide carboxylique comprenant au moins deux groupes, dont un est un groupe acide carboxylique et les autres peuvent être un groupe acide carboxylique ou un groupe hydroxyle ; ou un sel de celui-ci et
(iv) un support topiquement acceptable,
dans laquelle le ratio en mole du composé de titane à l'acide carboxylique se trouve dans l'intervalle de 1:0,01 à 1:5.

2. Composition selon la revendication 1, dans laquelle le composé de titane est le 2,2,2-nitrilotriséthanolate de titane (nombre CAS : 15879-01-3) présentant la structure suivante :

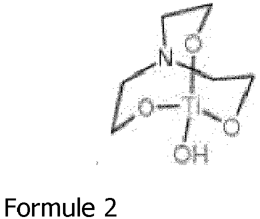

3. Composition selon la revendication 1 ou 2, dans laquelle l'acide minéral est choisi parmi l'acide chlorhydrique, l'acide nitrique, l'acide phosphorique ou l'acide sulfurique.

4. Composition selon l'une quelconque des revendications précédentes, dans laquelle l'acide minéral est inclus dans une quantité de 0,01 à 7 % en masse de la composition.

5. Composition selon l'une quelconque des revendications précédentes, dans laquelle l'acide carboxylique est choisi parmi l'acide oxalique, l'acide malonique, l'acide adipique, l'acide maléique, l'acide fumarique, l'acide glycolique, l'acide lactique, l'acide salicylique, l'acide citrique, l'acide tartarique, l'acide gluconique, ou l'acide malique.

6. Composition selon la revendication 5, dans laquelle l'acide carboxylique est choisi parmi l'acide tartarique, l'acide oxalique, l'acide lactique ou l'acide citrique.

7. Composition selon l'une quelconque des revendications précédentes, dans laquelle le composé de titane est inclus dans de 1 à 30 % en masse de la composition.

8. Composition selon l'une quelconque des revendications précédentes, dans laquelle le ratio en mole du composé de titane à l'acide minéral se trouve dans l'intervalle de 1:0,1 à 1:10.

9. Composition selon l'une quelconque des revendications précédentes, dans laquelle le support topiquement acceptable comprend un support hydrophobe ou un support aqueux.

10. Composition selon la revendication 9, dans laquelle le support hydrophobe comprend un composé de silicone, alcool de faible point d'ébullition ou une cire.

11. Composition selon la revendication 9 ou 10 qui est délivrée comme une bille, un solide ferme ou un stick.

12. Composition selon l'une quelconque des revendications 1 à 8 précédentes, dans laquelle le support topiquement acceptable comprend un gaz propulseur et la composition est délivrée comme un aérosol.

13. Procédé de réduction de transpiration comprenant l'étape d'application d'une composition selon l'une quelconque des revendications précédentes sur la surface de peau souhaitée.

14. Utilisation non-thérapeutique d'un composé de titane chélaté par une alcanolamine présentant la formule chimique :
TiNₐ(NH)_{b}(NH₂)_{c}O_{d}(OH)ₑ(CH₂)ₘ(CH₃)ₙ Formule 1
où a, b et c présentent chacun indépendamment une valeur de 0 à 3 ;
d et e présentent chacun indépendamment une valeur de 0 à 10 ;
m présente une valeur de 1 à 18 ;
n présente une valeur de 0 à 9 ;
dans laquelle a+b+c présente une valeur de 1 à 3 ; d+e présente une valeur de 4 à 10 et m+n présente une valeur de 1 à 18,
comme un actif antiperspirant.
